(19) Europäisches Patentamt
European Patent Office
Office européen des brevets

(11) **EP 4 365 276 A1**

(12) **EUROPEAN PATENT APPLICATION**

(43) Date of publication:
**08.05.2024 Bulletin 2024/19**

(21) Application number: **23206144.0**

(22) Date of filing: **26.10.2023**

(51) International Patent Classification (IPC):
**C12M 1/32** (2006.01)     **C12M 1/12** (2006.01)
**C12M 1/30** (2006.01)

(52) Cooperative Patent Classification (CPC):
**C12M 33/02; C12M 23/12; C12M 25/00;
C12M 25/01**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.11.2022 JP 2022176681**

(71) Applicant: **FUJIFILM Corporation
Tokyo 106-8620 (JP)**

(72) Inventors:
• **KASAI, Yusuke
Kanagawa, 258-8577 (JP)**
• **OBA, Takahiro
Kanagawa, 258-8577 (JP)**

(74) Representative: **Klunker IP
Patentanwälte PartG mbB
Destouchesstraße 68
80796 München (DE)**

(54) **PATTERN FORMING DEVICE AND PATTERN FORMING METHOD**

(57)     The pattern forming device includes at least one columnar structure body having a holding surface for holding a liquid at a tip thereof. The pattern forming method includes forming liquid droplets of a cell suspension or a cell adhesive on the holding surface and making the liquid droplets contact with the culture surface.

FIG. 1

EP 4 365 276 A1

**Description**

**BACKGROUND OF THE INVENTION**

1. Field of the Invention

The disclosed technology relates to a pattern forming device and a pattern forming method.

2. Description of the Related Art

The following techniques are known as techniques for patterning cells on a substrate. JP2014-168450A describes a manufacturing method for a substrate for a cell array, including a step of bringing a cell membrane modifier solution into contact with an end surface of a columnar body of a pillar array having a plurality of columnar bodies having substantially the same length on one main surface; and a step of positioning the pillar array on one main surface of a substrate so that the end surfaces of the columnar bodies face each other and transferring the cell membrane modifier solution that has adhered to the end surface, to the one main surface of the substrate.

JP2008-079517A describes that a leg part of a pin-shaped member of a replicator is caused to enter each well of a microplate, a culture supernatant in the well is collected, and the culture supernatant is moved to a well of another plate.

JP2020-195385A describes that a position of an instrument including a plurality of pins is aligned with a position of a chip having a plurality of wells, the instrument is lowered such that the pins are immersed in a sample in the well, and the sample that has adhered to the pin is transferred to a new chip.

**SUMMARY OF THE INVENTION**

Pattern culture is a technique for culturing cells to form a pattern having a desired shape and size on a culture base material. According to the pattern culture, it is possible to evaluate the cell population for each compartmented constitutional unit while minimizing the number of cells required. This makes it possible to efficiently quantify and standardize the state of cells, and it is possible to stabilize the evaluation system. The development of the pattern culture technique is expected to lead to the development in the field of life science such as drug discovery screening and regenerative medicine, in which cells are treated. It is known that cells exhibit functions while forming colonies to be matured. As a result, there is a demand for a pattern culture technique that makes it possible to form a large-sized pattern of about several hundred to several thousand $\mu$m for various cells.

The disclosed technology aims to form a pattern having a relatively large size in the pattern culture.

A pattern forming device according to the disclosed technology is a device for forming a cell pattern or a cell adhesive pattern on a culture surface of a culture base material. The cells include cell aggregates such as colonies in which a plurality of cells are aggregated. The pattern forming device includes at least one columnar structure body having at least one holding surface for holding a liquid at a tip thereof. The liquid held on the holding surface is typically a cell suspension or a cell adhesive.

The holding surface may be made of a material having weakly hydrophilic or hydrophobicity. The holding surface may be made of a material having a contact angle of 45° or more, where the contact angle is formed between the holding surface and water. The holding surface may be made of a resin. The holding surface may be made of a material that is resistant to autoclaving processes.

The columnar structure body may have at least one side surface, where an angle formed between the side surface and the holding surface is 5° or more and 175° or less. The columnar structure body may have a side surface, where an angle formed between the side surface and the holding surface is less than 90°.

The pattern forming device may further include a member for adjusting a distance between the holding surface and the culture surface, where the member is at least one adjustment structure that is integrated with a columnar structure body. The pattern forming device may further include a member for adjusting a distance between the holding surface and the culture surface, where the member is at least one adjustment structure that has a through-hole into which a columnar structure body is inserted.

The pattern forming device may further include a member for determining a relative position between the culture base material and the columnar structure body in a direction parallel to the culture surface, where the member is at least one position adjustment part that is integrated with a columnar structure body. The pattern forming device may further include a member for determining a relative position between the culture base material and the columnar structure body in a direction parallel to the culture surface, where the member is at least one position adjustment part that has a through-hole into which a columnar structure body is inserted. The center line of the position adjustment part may coincide with the center line of the columnar structure body. The pattern forming device may have a gap of 0.05 mm or more and 5 mm or less with respect to the culture substrate, where the gap is connected from the culture surface to the atmosphere. The columnar structure body may be surrounded by a bank structure.

The pattern forming device may include a substrate and a plurality of columnar structure bodies arranged on a surface of the substrate.

The pattern forming method according to the disclosed technology is a method of forming a cell pattern or a cell adhesive pattern on the culture surface by using the above-described pattern forming device, where the pattern forming method includes forming a liquid droplet of a cell suspension or a cell adhesive on the holding surface and making the liquid droplet contact with the culture surface.

The liquid droplet may be formed by dropping the cell suspension or the cell adhesive onto the holding surface in a state where the holding surface faces upward in a vertical direction. The liquid droplet may be formed so that a gas-liquid-solid interface is formed on an outer edge of the holding surface. The liquid droplet may be contacted with the culture surface in the state where the holding surface on which the liquid droplet has been formed faces upward in the vertical direction.

A size of a cell pattern or a cell adhesive pattern, which is formed on the culture surface, may be controlled by the amount of the liquid droplet. The size of a cell pattern or a cell adhesive pattern, which is formed on the culture surface, may be controlled by a distance between the holding surface and the culture surface in a case where the liquid droplet is contacted with the culture surface.

The culture surface may be a bottom surface of each of a plurality of wells provided in a well plate, and the pattern forming device may have a plurality of the columnar structure bodies arranged to match with the plurality of wells. A liquid droplet of a cell suspension or a cell adhesive may be formed on the holding surface of each of the columnar structure bodies, and each of the liquid droplets may be contacted with the bottom surface of each of the plurality of wells in a batch treatment.

A liquid droplet of the cell adhesive may be formed on the holding surface, the liquid droplet of the cell adhesive may be contacted with the culture surface, cells may be seeded on the culture surface, and the cells may be cultured on the culture surface.

According to the disclosed technology, it is possible to form a pattern having a relatively large size in the pattern culture.

**BRIEF DESCRIPTION OF THE DRAWINGS**

Fig. 1 is a perspective view showing an example of a configuration of a pattern forming device according to the embodiment of the disclosed technology.
Fig. 2 is a cross-sectional view showing an example of a configuration of a tip part of a columnar structure body according to the embodiment of the disclosed technology.
Fig. 3 is a cross-sectional view showing a state where a liquid is held on a holding surface according to the embodiment of the disclosed technology.
Fig. 4A is a view showing an example of a method of forming a cell pattern on a culture surface of a culture base material by using the pattern forming device according to the embodiment of the disclosed technology.
Fig. 4B is a view showing an example of the method of forming a cell pattern on a culture surface of a culture base material by using the pattern forming device according to the embodiment of the disclosed technology.
Fig. 4C is a view showing an example of the method of forming a cell pattern on a culture surface of a culture base material by using the pattern forming device according to the embodiment of the disclosed technology.
Fig. 4D is a view showing an example of the method of forming a cell pattern on a culture surface of a culture base material by using the pattern forming device according to the embodiment of the disclosed technology.
Fig. 4E is a view showing an example of the method of forming a cell pattern on a culture surface of a culture base material by using the pattern forming device according to the embodiment of the disclosed technology.
Fig. 4F is a view showing an example of the method of forming a cell pattern on a culture surface of a culture base material by using the pattern forming device according to the embodiment of the disclosed technology.
Fig. 4G is a view showing an example of the method of forming a cell pattern on a culture surface of a culture base material by using the pattern forming device according to the embodiment of the disclosed technology.
Fig. 5A is a view showing another example of the method of forming a cell pattern on a culture surface of a culture base material by using the pattern forming device according to the embodiment of the disclosed technology.
Fig. 5B is a view showing another example of the method of forming a cell pattern on a culture surface of a culture base material by using the pattern forming device according to the embodiment of the disclosed technology.
Fig. 6 is a view showing another example of the method of forming a cell pattern on a culture surface of a culture base material by using the pattern forming device according to the embodiment of the disclosed technology.
Fig. 7A is a perspective view showing an example of a configuration of a distance adjustment part according to the embodiment of the disclosed technology.
Fig. 7B is a cross-sectional view of a distance adjustment part according to the embodiment of the disclosed technology.
Fig. 8 is a cross-sectional view showing an example of an aspect of adjustment of a distance between a holding surface and a culture surface by the distance adjustment part according to the embodiment of the disclosed technology.

Fig. 9 is a plan view showing an example of a configuration of the pattern forming device according to the embodiment of the disclosed technology.

Fig. 10A is a perspective view showing an example of a configuration of a position adjustment part according to the embodiment of the disclosed technology.

Fig. 10B is a cross-sectional view of the position adjustment part according to the embodiment of the disclosed technology.

Fig. 11 is a cross-sectional view showing an example of an aspect of adjustment of a relative position between a culture base material and a columnar structure body by the position adjustment part according to the embodiment of the disclosed technology.

Fig. 12 is a plan view showing an example of a configuration of the pattern forming device according to the embodiment of the disclosed technology.

Fig. 13 is a cross-sectional view showing an example of a configuration of a tip part of the columnar structure body according to the embodiment of the disclosed technology.

Fig. 14 is an image of a pattern forming device according to Example of the disclosed technology.

Fig. 15A is an image showing each liquid droplet held on a holding surface of a columnar structure body according to Example of the disclosed technology.

Fig. 15B is an enlarged image of a liquid droplet held on a holding surface of a columnar structure body according to Example of the disclosed technology.

Fig. 16 is a microscopic image showing a vitronectin pattern formed on a bottom surface of each of a plurality of wells according to Example of the disclosed technology.

Fig. 17 is a microscopic image showing a cell pattern formed on a bottom surface of each of a plurality of wells according to Example of the disclosed technology.

Fig. 18 is a perspective view showing an example of a configuration of a pattern forming device according to another embodiment of the disclosed technology.

Fig. 19 is a view showing an example of a method of forming a cell pattern on a culture surface of a culture base material by using the pattern forming device according to another embodiment of the disclosed technology.

## DESCRIPTION OF THE PREFERRED EMBODIMENTS

Hereinafter, one example of the embodiment of the disclosed technology will be described with reference to the drawings. It is noted that the same or equivalent components and portions in the drawings are assigned by the same reference numerals, and the overlapping description will be omitted.

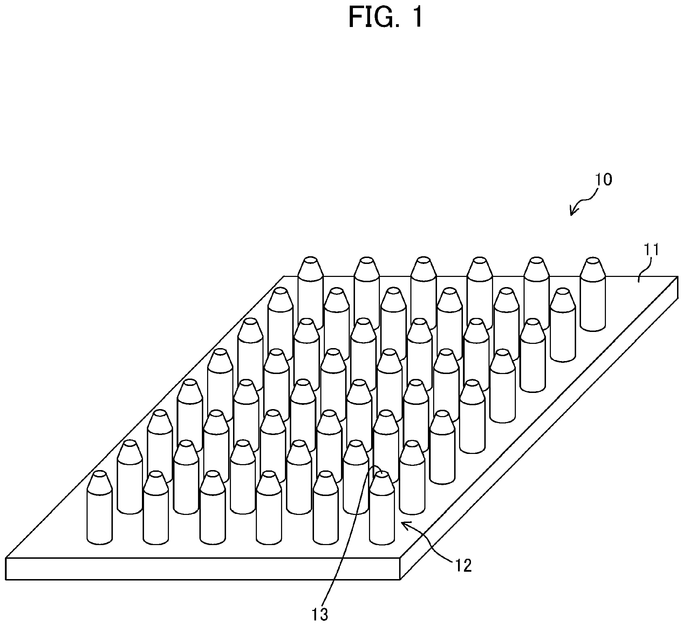

Fig. 1 is a perspective view showing an example of a configuration of a pattern forming device 10 according to the embodiment of the disclosed technology. The pattern forming device 10 is a device for forming a cell pattern or a cell adhesive pattern on a culture surface of a culture base material. The cells include cell aggregates such as colonies in which cells are aggregated. The pattern forming device 10 has a plurality of columnar structure bodies 12 arranged on a surface of a substrate 11. Each of the columnar structure bodies 12 has a columnar structure in which a direction perpendicular to the main surface of the substrate 11 is set as an axial direction. Although the length of the columnar structure body 12 in the axial direction is not particularly limited, it may be, for example, 1 mm or more and 100 mm or less.

The culture base material is a base material on which a cell pattern or a cell adhesive pattern is formed by the pattern forming device 10 and is a base material having a culture surface on which cells are cultured. The culture base material may be, for example, a well plate having a plurality of wells, and the culture surface may be, for example, a bottom surface of each of a plurality of wells provided in a well plate. The plurality of columnar structure bodies 12 may be provided to match with a plurality of wells provided in the well plate. Fig. 1 exemplarily shows a configuration in which forty-eight columnar structure bodies 12 provided to match with the respective wells in a well plate having 48 (6 × 8) wells are provided. The arrangement form (the number of rows and columns and the arrangement interval) of the columnar structure bodies 12 is not limited to the form exemplified in Fig. 1 and is appropriately determined according to the arrangement of the cell pattern to be formed on the culture base material.

Each of the columnar structure bodies 12 has a holding surface 13 for holding a liquid at the tip thereof. The liquid held on the holding surface 13 is typically a cell suspension or a cell adhesive. A pattern which is formed on the culture surface of the culture base material by the pattern forming device 10 reflects the planar shape of the holding surface 13. Fig. 1 exemplarily shows the holding surface 13 of which the planar shape is circular. The diameter of the holding surface 13 is not particularly limited, but may be, for example, 0.5 mm or more and 5 mm or less. The shape of the holding surface 13 is appropriately determined according to the cell pattern or the cell adhesive pattern to be formed on the culture base material.

In the columnar structure body 12, it is preferable that at least the holding surface 13 is made of a material having weakly hydrophilic or hydrophobicity. In a case where the holding surface 13 is made of a material having weakly hydrophilic or hydrophobicity, it is possible to increase the amount of liquid that is capable of being held in the holding surface 13. This makes it possible to form a cell pattern or a cell adhesive pattern having a relatively large size, on the culture surface of the culture base material. It is preferable that the holding surface 13 of the columnar structure body 12 is made of a material having a contact angle of 45° or more, where the contact angle is formed between the holding surface and water. In addition, it is preferable that the columnar structure body 12 has resistance to a high-pressure steam sterilization treatment, that is, heat resistance. In addition, it is preferable that the columnar structure 12 has resistance to gamma ray sterilization, electron beam sterilization, EOG sterilization, and ethanol sterilization. In the columnar structure body 12, at least the holding surface 13 may be made of a resin. As a material of the columnar structure body 12 (holding surface 13), it is possible to suitably use, for example, thermosetting resins such as PF, EP, PI and thermoplastic resins such as PVC, PVAC, PVAL, PC, PVB, PS, ABS, PMMA, PPO, PE, PS, PP, PA, POM, PPS, PVDC, PEEK, PTFE. The columnar structure body 12 may be made of the same material as the substrate 11 and may be integrated with the substrate 11. The columnar structure 12 may be made by cutting or injection molding.

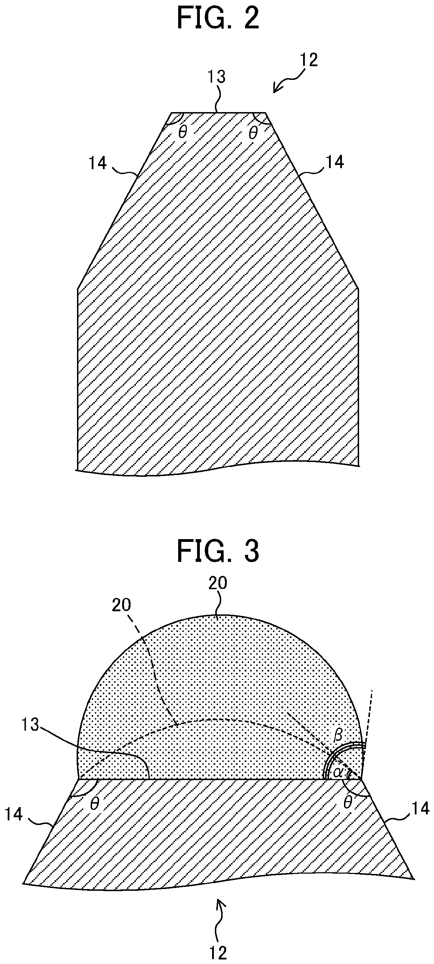

Fig. 2 is a cross-sectional view showing an example of a configuration of a tip part of the columnar structure body 12. The columnar structure body 12 has a side surface 14, where the angle θ formed between the side surface 14 and the holding surface 13 is an obtuse angle (that is, larger than 90°). That is, the tip part of the columnar structure body 12 has a truncated cone shape having the holding surface 13 as the upper surface, and the cross section of the columnar structure body 12, which is orthogonal to the axial direction, gradually decreases toward the holding surface 13. A curvature radius of a corner portion formed between the holding surface 13 and the side surface 14 is not particularly limited; however, it may be, for example, 0.01 $\mu$m or more and 100 $\mu$m or less.

Fig. 3 is a cross-sectional view showing a state where a liquid is held on the holding surface 13. In a case of dropping a liquid onto the holding surface 13, a liquid droplet 20 is formed on the holding surface 13. The maximum value β of the angle formed between the holding surface 13 and the tangent line of the liquid droplet 20 is represented according to Expression (1). In Expression (1), α is a contact angle between the liquid dropped onto the holding surface 13 and the holding surface 13. The larger the β is, the larger the amount of liquid capable of being held in the holding surface 13 is. As a result, the smaller the angle θ formed between the holding surface 13 and the side surface 14 is, the larger the amount of liquid capable of being held in the holding surface 13 can be.

$$\beta = \alpha + 180° - \theta \cdots (1)$$

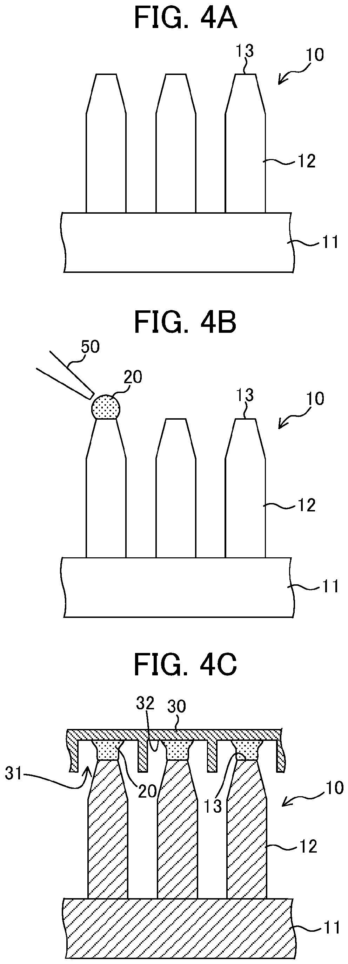

Hereinafter, a method of forming a cell pattern or a cell adhesive pattern on a culture surface of a culture base material by using the pattern forming device 10 will be described with reference to Figs. 4A to 4G. First, the pattern forming device 10 is installed so that the holding surface 13 of the columnar structure body 12 faces upward in the vertical direction. In this case, the holding surface 13 is horizontal (Fig. 4A).

Next, a cell suspension or a cell adhesive is dropped onto the holding surface 13 of each of the columnar structure bodies 12 in a state where the holding surface 13 faces upward in the vertical direction, whereby the liquid droplet 20 of the cell suspension or the cell adhesive is formed so that a gas-liquid-solid interface is formed on the outer edge of the holding surface 13 (Fig. 4B). The dropping of the cell suspension or the cell adhesive onto the holding surface 13 can be carried out using, for example, a micropipette 50.

It is possible to control the size of the cell pattern or the cell adhesive pattern, which is formed on the culture surface of the culture base material, by the amount of the liquid droplet 20 formed on the holding surface 13. The amount of the liquid droplet 20 formed on the holding surface 13 is determined according to the size of the pattern to be formed on a culture surface 32. In a case of carrying out the formation of the liquid droplet 20 by dropping a liquid onto the holding surface 13, it is possible to exhibit a "pinning effect" that holds the liquid droplet 20 on the holding surface 13. Due to the "pinning effect", it is possible to hold the liquid droplet on the holding surface 13, where the contour of the liquid droplet has spread to the outside of the outer edge of the holding surface 13. In addition, due to the "pinning effect", it is possible to stabilize the state of the liquid droplet 20 formed on the holding surface 13. This makes it possible to make uniform the state of the liquid droplet 20 formed on the holding surface 13 thereof between a plurality of columnar structure bodies 12, and consequently, it is possible to make uniform the cell pattern or the cell adhesive pattern, which is formed on the culture surface of the culture base material.

Next, the liquid droplet 20 is contacted with the culture surface 32 of a culture base material 30 in a state where the holding surface 13 on which the liquid droplet 20 has been formed faces upward in the vertical direction (Fig. 4C). That is, the columnar structure body 12 is covered from above with culture base material 30, whereby the liquid droplet 20 is contacted with the culture surface 32. As a result, the cell suspension or the cell adhesive, which is held on the holding surface 13, adheres to the culture surface 32 of the culture base material 30. The culture base material 30 may be a well plate having a plurality of wells 31. In this case, a bottom surface of each of wells 31 is the culture surface 32. Each of the plurality of columnar structure bodies 12 provided in the pattern forming device 10 matches with each of a plurality of wells 31 provided in the culture base material 30.

In a case where the liquid droplet 20 formed on the holding surface 13 is contacted with the culture surface 32, the position of the columnar structure body 12 is aligned with the position of the well 31 in the horizontal direction in a state where the bottom surface of the well 31 faces downward. Thereafter, the culture surface 32 is gradually brought close to the holding surface 13 while maintaining the culture surface 32 horizontally, and thereafter, each liquid droplet 20 formed on the holding surface 13 of each of the plurality of columnar structure bodies 12 is contacted with the bottom surface (the culture surface 32) of each of the plurality of wells 31 in a batch treatment.

It is possible to control a size of a pattern which is formed on the culture surface 32 by a distance between the holding surface 13 and the culture surface 32 in a case where the liquid droplet 20 is contacted with the culture surface 32. The distance between the holding surface 13 and the culture surface 32 in a case where the liquid droplet 20 is contacted with the culture surface 32 is determined according to a size of a pattern to be formed on the culture surface 32.

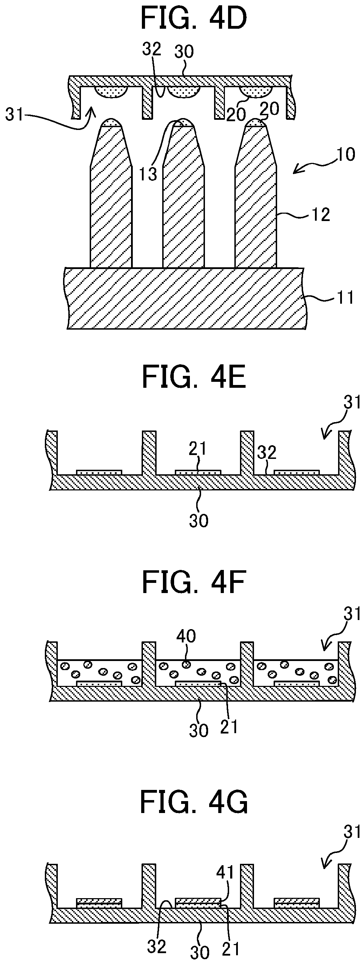

After the liquid droplet 20 is contacted with the culture surface 32, the culture surface 32 is moved away from the holding surface 13. As a result, the liquid droplet 20 is split into the holding surface 13 side and the culture surface 32 side (Fig. 4D). A cell suspension pattern or a cell adhesive pattern, which reflects the planar shape of the holding surface 13, is formed on the bottom surface (culture surface 32) of each of the plurality of wells 31 by a batch treatment. Hereinafter, a case where a cell adhesive pattern is formed on the bottom surface (culture surface 32) of each well 31 will be described as an example. As a cell adhesive, it is possible to use an extracellular matrix protein such as vitronectin, laminin, fibronectin, collagens, or gelatins.

After a cell adhesive 21 is allowed to adhere to the culture surface 32, the excess of the cell adhesive 21 in each well 31 is removed. The cell adhesive 21 that has adhered to the bottom surface (the culture surface 32) of each well 31 remains on the culture surface 32 while the pattern is maintained (Fig. 4E).

Next, cells 40 are seeded on the culture surface 32 (Fig. 4F). The seeding of the cells 40 is carried out by injecting a cell suspension adjusted to a predetermined cell concentration into each well 31. The cell 40 may be any adhesive cell, and the kind of the cells 40 is not particularly limited. The cell 40 may be, for example, a human-derived induced pluripotent stem cell (iPS cell).

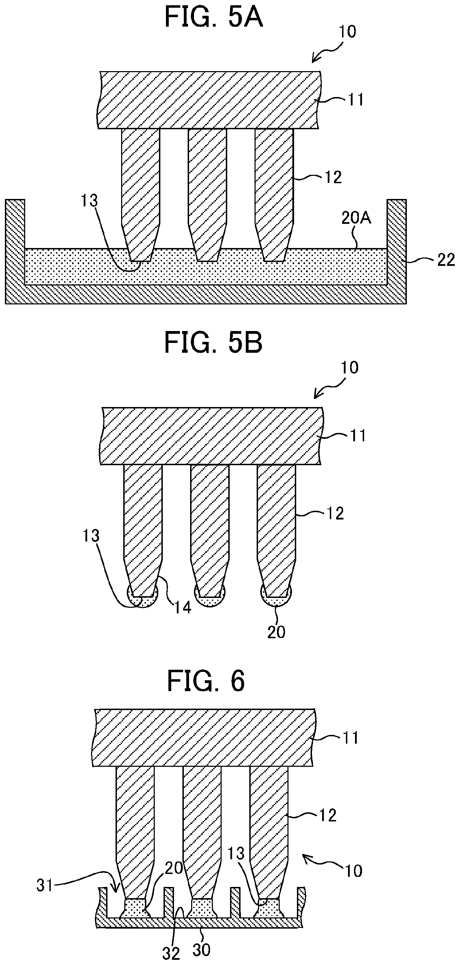

The cell 40 adheres to a region coated with the cell adhesive 21 on the culture surface 32. Thereafter, the cell 40 is cultured in a suitable environment, whereby the cell 40 proliferates in the region coated with the cell adhesive 21. As a result, a cell pattern 41 corresponding to the pattern of the cell adhesive 21 is formed on the culture surface 32 (Fig. 4G). It is noted that the liquid droplet 20 of the cell suspension may be formed on the holding surface 13 of the columnar structure body 12, and the liquid droplet 20 of the cell suspension may be contacted with the culture surface 32 to form a cell suspension pattern on the culture surface 32. In addition, in a case where cells or a cell adhesive is allowed to stand to adhere to the culture surface 32, it may be allowed to stand in any one state of Fig. 4C, Fig. 4D, or Fig. 6. In a case where the liquid droplet 20 of the cell suspension is contacted with the culture surface 32, it is preferably allowed to stand in the state of Fig. 6.

According to the pattern forming device 10 and the pattern forming method according to the embodiments of the disclosed technology, it is possible to form a pattern having a size corresponding to the size of the liquid droplet 20 formed on the holding surface 13, and thus it is possible to form a pattern having a relatively large size.

It is noted that in the above description, a case where the liquid droplet 20 is formed on the holding surface 13 by dropping a cell suspension or a cell adhesive onto the holding surface 13 of the columnar structure body 12 has been exemplified (Fig. 4B). However, the disclosed technology is not limited to this aspect. For example, as shown in Fig. 5A, the liquid droplet 20 of the cell suspension or the cell adhesive may be formed on the holding surface 13 of the columnar structure body 12, as shown in Fig. 5B, by immersing the tip part of the columnar structure body 12 in a liquid 20A which is a cell suspension or a cell adhesive stored in a container 22. According to this aspect, it is possible to form the liquid droplet 20 on each of the plurality of columnar structure bodies 12 in a batch treatment, and it is possible to increase a treatment rate. However, in this case, it is not possible to exhibit a "pinning effect" that holds the liquid droplet 20 on the holding surface 13 since the side surface 14 in the periphery of the holding surface 13 is wetted by the liquid 20A, and thus the liquid droplet 20 formed on the holding surface 13 moves easily along the axial direction of the columnar structure body 12. In this case, the state of the liquid droplet 20 formed on the holding surface 13 is not stable, and thus variations occur in the state of the liquid droplet 20 formed on the holding surface 13 between the plurality of columnar structure bodies 12. As a result, there is a concern that the shape and the size of the pattern which is formed on the culture surface of the culture base material may be non-uniform.

On the other hand, as shown in Fig. 4B, in a case where liquid droplet 20 is formed on the holding surface 13 by dropping a cell suspension or a cell adhesive onto the holding surface 13 of the columnar structure body 12, the side surface 14 in the periphery of the holding surface 13 is not wetted, and thus it is not possible to exhibit a "pinning effect" that holds the liquid droplet 20 on the holding surface 13, that makes it possible to stabilize the state of the liquid droplet 20 formed on the holding surface 13. As a result, it is possible to make the state of the liquid droplet 20 formed on the holding surface 13 uniform between a plurality of columnar structure bodies 12, and consequently, it is possible to make uniform the pattern which is formed on the culture surface of the culture base material.

In addition, in the above description, a case where the liquid droplet 20 is contacted with the culture surface 32 in a state where the holding surface 13 on which the liquid droplet 20 has been formed faces upward in the vertical direction has been exemplified (Fig. 4C).

However, the disclosed technology is not limited to this aspect. As shown in Fig. 6, the liquid droplet 20 may be contacted with the culture surface 32 of the culture base material 30 in a state where the holding surface 13 on which the liquid droplet 20 has been formed faces downward in the vertical direction. That is, the liquid droplet 20 may be contacted with the culture surface 32 by lowering the pattern forming device 10 from above, onto the culture base material 30 that has been allowed to stand. However, in this case, a force acts on the liquid droplet 20, due to gravity, in a direction away from the holding surface 13, and thus the amount of the liquid that is capable of being held on the holding surface 13 is reduced as compared with a case where the holding surface 13 faces upward in the vertical direction. In addition, since the pattern forming device 10 is lowered in a state where the liquid droplet 20 has been formed on the holding surface 13, there is a concern that the liquid droplet 20 may fall off from the holding surface 13 due to vibration. That is, in the aspect shown in Fig. 6, it is difficult to handle the liquid droplet 20.

On the other hand, as shown in Fig. 4C, in a case of making the liquid droplet 20 contact with the culture surface 32 in a state where the holding surface 13 faces upward in the vertical direction, a state where the liquid droplet 20 is supported by the holding surface 13 is maintained, and thus it is possible to increase the amount of liquid that is capable of being held in the holding surface 13 as compared with a case where the holding surface 13 faces downward in the vertical direction. In addition, since the pattern forming device 10 can be kept in a stationary state, it is possible to avoid falling off the liquid droplets 20 from the holding surface 13.

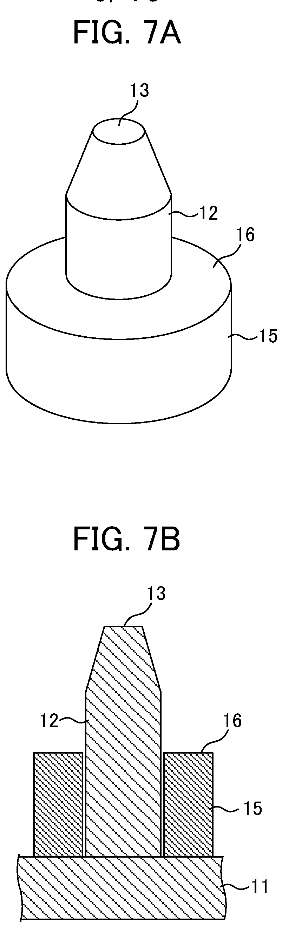

The pattern forming device 10 may include a member for adjusting a distance between the holding surface 13 and the culture surface 32 in a case of making the liquid droplet 20 contact with the culture surface 32. Fig. 7A is a perspective view showing an example of a configuration of a distance adjustment part 15 which is a member for adjusting the distance between the holding surface 13 and the culture surface 32, and Fig. 7B is a cross-sectional view of the distance adjustment part 15. The distance adjustment part 15 may be a member having an annular structure having a through-hole into which the columnar structure body 12 is inserted. The shape of the distance adjustment part 15 may be a cylindrical shape. The distance adjustment part 15 has a support surface 16 parallel to the holding surface 13 of the columnar structure body 12. The distance adjustment part 15 may be constituted as a member separated from the columnar structure body 12 or may be attachably and detachably provided on the columnar structure body 12. In addition, the distance adjustment part 15 may be provided to be integrated with the columnar structure body 12. In other words, the columnar structure body 12 may have a stepped structure and have a part that functions as the distance adjustment part 15. In addition, the distance adjustment part 15 may be provided to be integrated with the substrate 11.

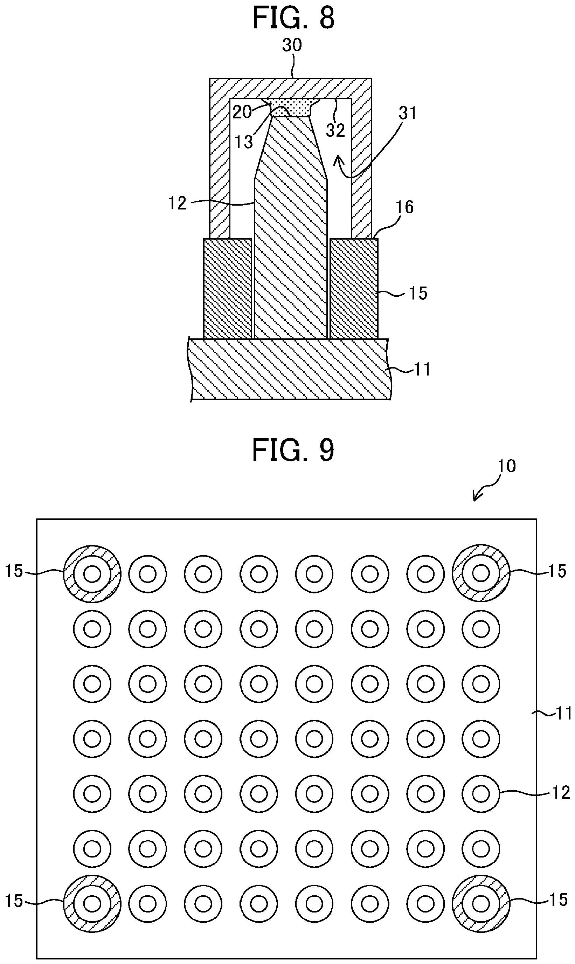

Fig. 8 is a cross-sectional view showing an example of an aspect of adjustment of the distance between the holding surface 13 and the culture surface 32 by the distance adjustment part 15. In a case where the culture surface 32 is contacted with the liquid droplet 20, the columnar structure body 12 is covered with the culture base material 30 from above. In this case, the tip part of the columnar structure body 12 enters the inside of the well 31 provided in the culture base material 30. The outer peripheral lower end part of the well 31 comes into contact with the support surface 16 of the distance adjustment part 15, and thus the movement of the culture base material 30 is restricted in the axial direction (downward direction) of the columnar structure body 12. The outer peripheral lower end part of the well 31 is supported by the support surface 16 of the distance adjustment part 15, and thus the distance between the holding surface 13 and the culture surface 32 is kept constant. This makes it possible to increase the uniformity of the pattern which is formed on the culture surface 32.

It is noted that as shown in Fig. 9, the distance adjustment part 15 may be provided for a part of the columnar structure bodies 12 among the plurality of columnar structure bodies 12 arranged on the surface of the substrate 11. Fig. 9 exemplarily shows a configuration in which the distance adjustment part 15 is provided for each of the four columnar structure bodies 12 disposed in the vicinity of the four corners of the substrate 11.

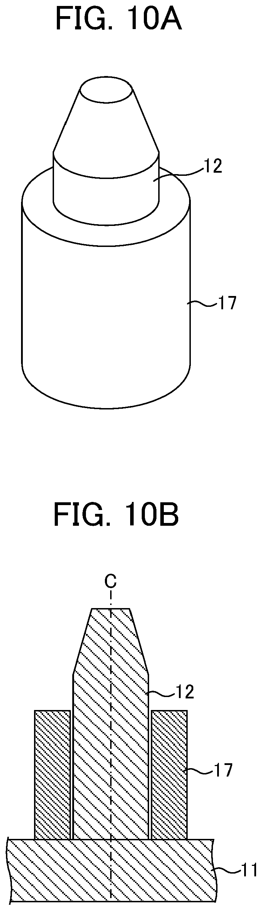

The pattern forming device 10 may include a member for determining, in a direction parallel to the culture surface 32, a relative position between the culture base material 30 and the columnar structure body 12 in a case of making the liquid droplet 20 contact with the culture surface 32. Fig. 10A is a perspective view showing an example of a configuration of the position adjustment part 17 which is a member for determining a relative position between the culture base material 30 and the columnar structure body 12 in a direction parallel to the culture surface 32, and Fig. 10B is a cross-sectional view of the position adjustment part 17. The position adjustment part 17 may be a member having an annular structure having a through-hole into which the columnar structure body 12 is inserted. The shape of the position adjustment part 17 may be a cylindrical shape. The center line C of the position adjustment part 17 may coincide with the center line of the columnar structure body 12. The position adjustment part 17 may be constituted as a member separated from the columnar structure body 12 or may be attachably and detachably provided on the columnar structure body 12. In addition, the position adjustment part 17 may be provided to be integrated with the columnar structure body 12. In other words, the columnar structure body 12 may have a stepped structure and have a part that functions as the position adjustment part 17. In addition, the position adjustment part 17 may be provided to be integrated with the substrate 11.

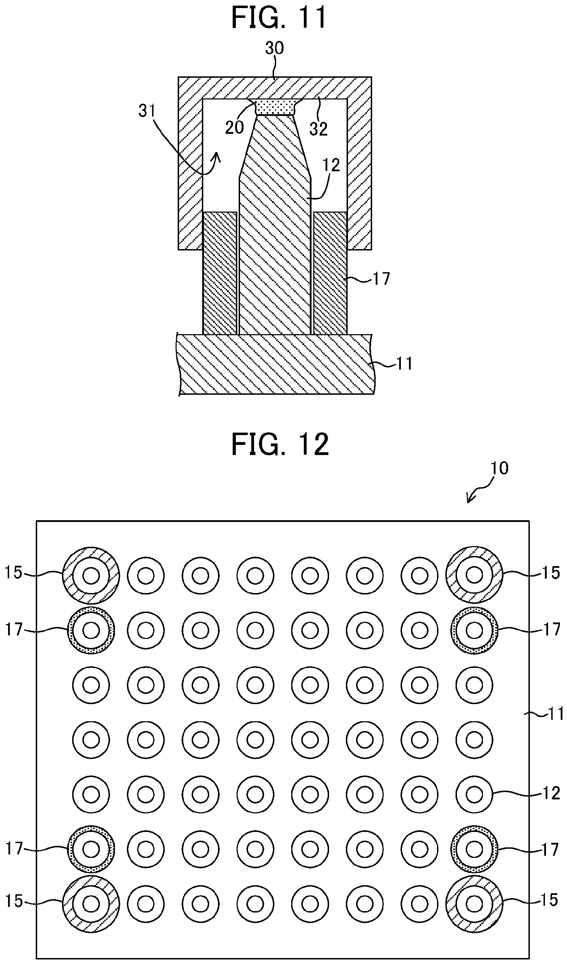

Fig. 11 is a cross-sectional view showing an example of an aspect of adjustment of a relative position between the culture base material 30 and the columnar structure body 12 by the position adjustment part 17. In a case where the culture surface 32 is contacted with the liquid droplet 20, the columnar structure body 12 is covered with the culture base material 30 from above. In this case, the tip part of the columnar structure body 12 enters the inside of the well 31 provided in the culture base material 30. The diameter of the position adjustment part 17 is slightly smaller than the diameter of the well 31, and thus the tip part of the position adjustment part 17 also enters the inside of the well 31. The interior wall of the well 31 comes into contact with the outer peripheral surface of the position adjustment part 17, and thus the movement of the culture base material 30 is restricted in the direction parallel to the culture surface 32. As a result, the relative position between the culture base material 30 and the columnar structure body 12 in the direction parallel to the culture surface 32 is determined. Since the center line of the position adjustment part 17 may coincide with the center line of the columnar structure body 12, it is possible to carry out the transfer of the liquid droplet 20 to the center of the bottom surface of the well 31 with high position accuracy.

It is noted that as shown in Fig. 12, the position adjustment part 17 may be provided for a part of the columnar structure bodies 12 among the plurality of columnar structure bodies 12 arranged on the surface of the substrate 11. In addition, since the pattern forming device 10 includes both the distance adjustment part 15 and the position adjustment part 17, it is possible to carry out, at the same time, the adjustment of the distance between the holding surface 13 and the culture surface 32 and the adjustment of the relative position between the culture base material 30 and the columnar structure body 12 in the direction parallel to the culture surface 32.

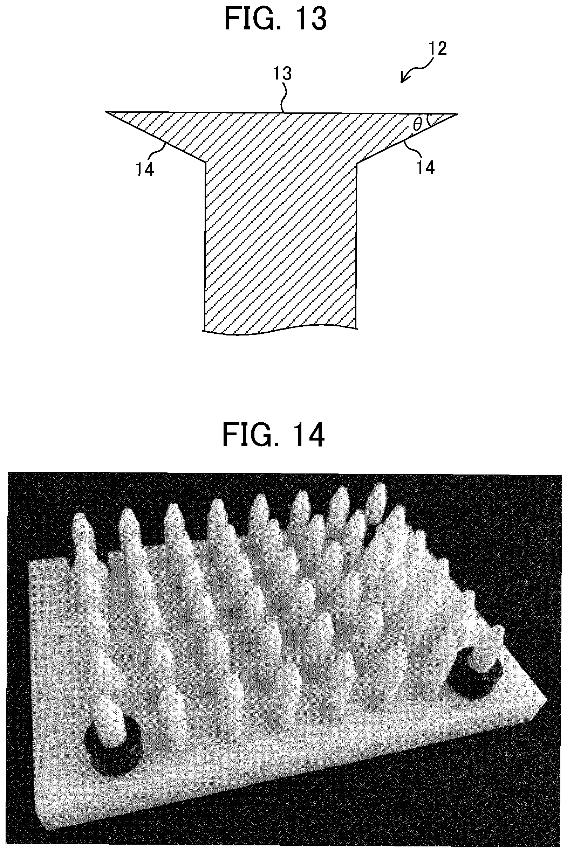

In addition, in the above description, the columnar structure body 12 in which the angle θ formed between the holding surface 13 and the side surface 14 is an obtuse angle (that is, larger than 90°) has been exemplified (Fig. 2). However, the disclosed technology is not limited to this aspect. As shown in Fig. 13, in the columnar structure body 12, the angle θ formed between the holding surface 13 and the side surface 14 may be an acute angle (that is, less than 90°). As shown in Expression (1), the maximum value β of the angle formed between the holding surface 13 and the tangent line of the liquid droplet 20 increases as the angle θ formed between the holding surface 13 and the side surface 14 decreases. That is, in a case where the angle θ formed between the holding surface 13 and the side surface 14 is set to an acute angle, β can be further increased, and thus it is possible to increase the size of the liquid droplet 20 formed on the holding surface 13, and consequently, it is possible to increase the size of the cell pattern or the cell suspension pattern which is to be formed on the culture surface 32. It is preferable to set the angle θ formed between the holding surface 13 and the side surface 14, for example, in a range of $5° \leq θ \leq 175°$ according to the size of the pattern to be formed on the culture surface 32.

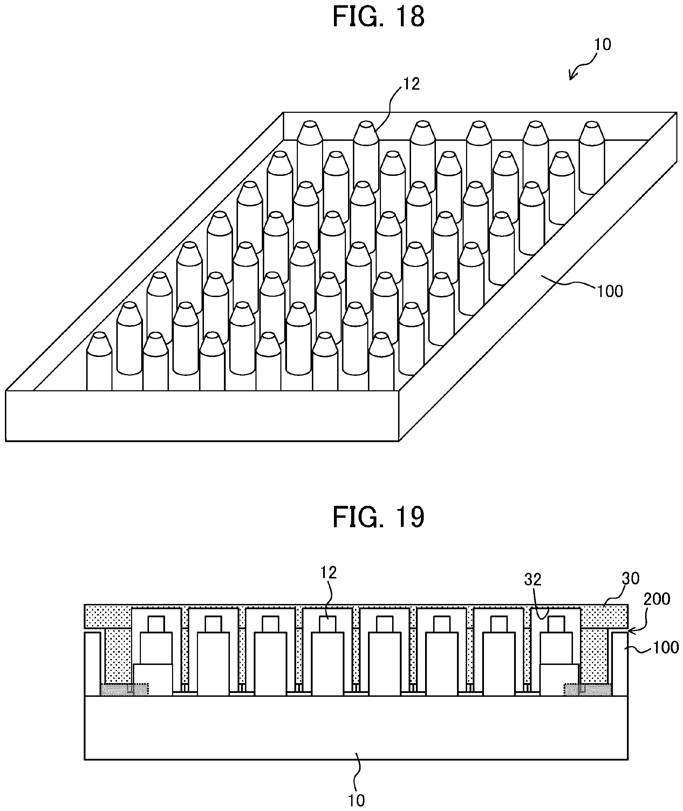

As shown in FIGS. 18 and 19, the pattern forming device 10 may have a bank structure 100 surrounding the columnar structure 12. Thereby, the risk of contamination can be reduced. Further, as shown in FIG. 19, the pattern forming device 10 may be configured such that a gap 200 of 0.05 mm or more and 5 mm or less is formed with respect to the culture substrate 30. The culture surface 32 is connected to the atmosphere via a gap 200. Since the gap 200 has a bent structure, invasion of bacteria can be suppressed.

Examples

Hereinafter, the disclosed technology will be described in more detail with reference to Examples. However, the disclosed technology is not limited to the following Examples.

Production of pattern forming device

A pattern forming device in which forty-eight columnar structure bodies (6 rows and 8 columns) were arranged on a substrate was produced. Fig. 14 shows an image of the produced pattern forming device. The tip part of the columnar structure body was formed in a truncated cone shape. Polyacetal was used as a material of the columnar structure body and the substrate. The diameter of the root portion of the columnar structure body is 6 mm, and the diameter of the holding surface of the tip part is 2.4 mm. The angle formed between the holding surface and the side surface is 109°. The length of the columnar structure body in the axial direction is 20 mm, and the interval between the columnar structure bodies is 13.08 mm. A distance adjustment part was attached to each of the four columnar structure bodies disposed in the vicinity of the four corners of the substrate. A culture base material is positioned by the distance adjustment part such that the distance between the holding surface and the culture surface is 1 mm. In addition, a position adjustment part was attached to each of the four columnar structure bodies adjacent to the columnar structure body to which the distance adjustment part had been attached. A relative position between each well of the culture base material and the columnar structure body in the plane direction and the height direction is uniquely determined by the position adjustment part and the distance adjustment part.

Formation of liquid droplet of cell adhesive

The pattern forming device was installed so that the holding surface of the columnar structure body faced upward in the vertical direction. In a state where the holding surface faced upward in the vertical direction, a cell adhesive was dropped onto the holding surface using a micropipette. 4 μL of a vitronectin solution having a con-

centration of 2.5 μg/mL was used as a cell adhesive. A liquid droplet of vitronectin was formed on the holding surface due to the "pinning effect".

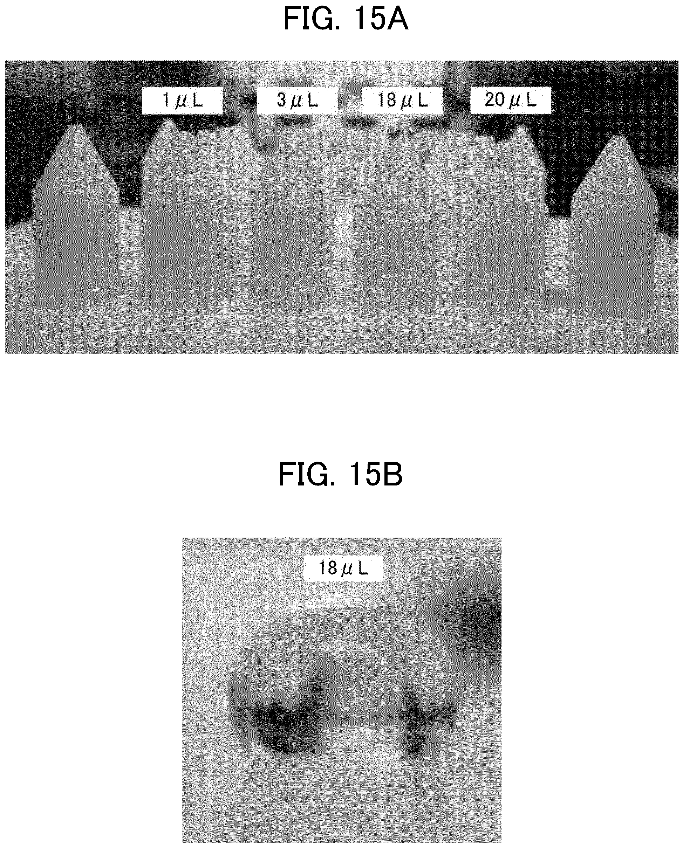

It was confirmed in advance that the "pinning effect" was exhibited in the produced pattern forming device. Each of 1 μL, 3 μL, 18 μL, and 20 μL of colored vitronectin was dropped onto the holding surface of the columnar structure body. Fig. 15A shows an image of the liquid droplet of each amount which is formed on the holding surface. Each of liquid droplets of 1 μL, 3 μL, and 18 μL was held on the holding surface due to the "pinning effect". Fig. 15B is an enlarged image of the liquid droplet having a liquid amount of 18 μL which is held on the holding surface. On the other hand, in a case where the liquid amount was set to 20 μL, the liquid droplet slid off from the holding surface.

Transfer of cell adhesive

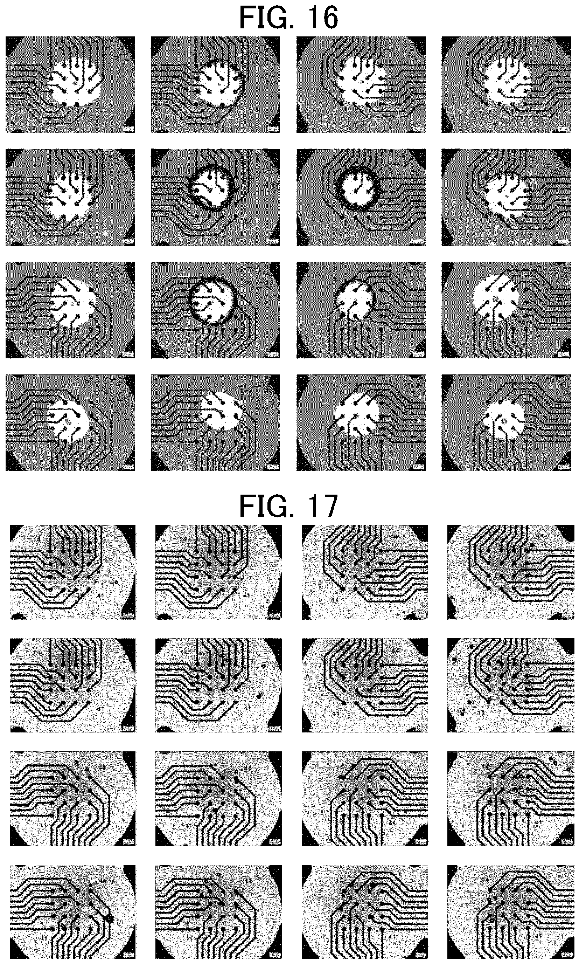

The columnar structure body was covered from above with a 48-well plate, whereby the liquid droplet of vitronectin held on the holding surface of the columnar structure body was contacted with the bottom surface of each well. Vitronectin was transferred to 16 wells in a batch treatment. Thereafter, the 48-well plate was removed from the pattern forming device and allowed to stand for 1 hour in order to fix the vitronectin in each well. Fig. 16 shows an image of a vitronectin pattern formed on the bottom surface of each well. In each well, a vitronectin pattern having a uniform shape, a uniform size, and a uniform arrangement was formed. Thereafter, the excess of the vitronectin in each well was removed. Next, in the part uncoated with vitronectin, a 3% bovine serum albumin solution was added to each well in order to inhibit non-specific cell adhesion.

Seeding and culturing of cells

A cell suspension containing human-derived iPS cells, in which the cell concentration was adjusted to $1 \times 10^5$ cells/ml, was injected into each of wells of a 48-well plate. E8 was used as a culture medium. The 48-well plate was accommodated in an incubator maintained in an environment of 37°C and a $CO_2$ concentration of 5%, and stationary culture was carried out for one day. Thereafter, the culture medium was exchanged to remove floating cells.

Fig. 17 shows an image of a cell pattern which is formed on the bottom surface of each well. Cells adhered to a region coated with vitronectin on the bottom surface of each well and proliferated in the region to form a cell pattern having a uniform shape, a uniform size, and a uniform arrangement in each well.

In regard to the embodiment described above, the following supplementary notes will be further disclosed.

Supplementary Note 1

A pattern forming device for forming a cell pattern or a cell adhesive pattern on a culture surface of a culture base material, the pattern forming device comprising:
at least one columnar structure body having at least one holding surface for holding a liquid at a tip thereof.

Supplementary Note 2

The pattern forming device according to Supplementary Note 1,
wherein the holding surface is made of a material having a contact angle of 45° or more, where the contact angle is formed between the holding surface and water.

Supplementary Note 3

The pattern forming device according to any one of Supplementary Note 1 or 2, wherein the holding surface is made of a resin.

Supplementary Note 4

The pattern forming device according to any one of Supplementary Notes 1 to 3,
wherein the holding surface is made of a material that is resistant to autoclaving processes.

Supplementary Note 5

The pattern forming device according to Supplementary Notes 1 to 4,
wherein the holding surface is made of a material having weakly hydrophobicity.

Supplementary Note 6

The pattern forming device according to any one of Supplementary Notes 1 to 5,
wherein the columnar structure body has at least one side surface, where an angle formed between the side surface and the holding surface is 5° or more and 175° or less.

Supplementary Note 7

The pattern forming device according to any one of Supplementary Notes 1 to 6,
wherein the columnar structure body has at least one side surface, where an angle formed between the side surface and the holding surface is less than 90°.

Supplementary Note 8

The pattern forming device according to any one of Supplementary Notes 1 to 7, further comprising:

at least one adjustment structure for adjusting a distance between the holding surface and the culture surface.

Supplementary Note 9

The pattern forming device according to Supplementary Note 8,
wherein the adjustment structure has a through-hole into which the columnar structure body is inserted.

Supplementary Note 10

The pattern forming device according to any one of Supplementary Notes 1 to 9, further comprising:
at least one position adjustment part for determining a relative position between the culture base material and the columnar structure body in a direction parallel to the culture surface.

Supplementary Note 11

The pattern forming device according to Supplementary Note 10,
wherein the position adjustment part has a through-hole into which the columnar structure body is inserted.

Supplementary Note 12

The pattern forming device according to Supplementary Note 10 or 11,
wherein a center line of the position adjustment part coincides with a center line of the columnar structure body.

Supplementary Note 13

The pattern forming device according to Supplementary Notes 1 to 12

wherein having a gap of 0.05 mm or more and 5 mm or less with respect to the culture substrate,
the gap is connected from the culture surface to the atmosphere.

Supplementary Note 14

The pattern forming device according to Supplementary Notes 1 to 13
wherein the columnar structure body is surrounded by a bank structure.

Supplementary Note 15

The pattern forming device according to any one of Supplementary Notes 1 to 14, further comprising:

a substrate; and
a plurality of the columnar structure bodies arranged on a surface of the substrate.

Supplementary Note 16

A pattern forming method of forming the cell pattern or the cell adhesive pattern on the culture surface by using the pattern forming device according to any one of Supplementary Notes 1 to 15, the pattern forming method comprising:

forming a liquid droplet of a cell suspension or a cell adhesive on the holding surface; and
making the liquid droplet contact with the culture surface.

Supplementary Note 17

The pattern forming method according to Supplementary Note 16,
wherein the liquid droplet is formed by dropping the cell suspension or the cell adhesive onto the holding surface in a state where the holding surface faces upward in a vertical direction.

Supplementary Note 18

The pattern forming method according to Supplementary Note 16 or 17,
wherein the liquid droplet is formed so that a gas-liquid-solid interface is formed on an outer edge of the holding surface.

Supplementary Note 19

The pattern forming method according to Supplementary Note 17 or 18,
wherein the liquid droplet is contacted with the culture surface in the state where the holding surface on which the liquid droplet is formed faces upward in the vertical direction.

Supplementary Note 20

The pattern forming method according to any one of Supplementary Notes 16 to 19,
wherein a size of the cell pattern or the cell adhesive pattern, which is formed on the culture surface, is controlled by an amount of the liquid droplet.

Supplementary Note 21

The pattern forming method according to any one of Supplementary Notes 16 to 20,
wherein a size of the cell pattern or the cell adhesive pattern, which is formed on the culture surface, is controlled by a distance between the holding surface and the culture surface in a case where the liquid droplet is contacted with the culture surface.

Supplementary Note 22

The pattern forming method according to any one of Supplementary Notes 16 to 21,

> wherein the culture surface is a bottom surface of each of a plurality of wells provided in a well plate, the pattern forming device has a plurality of the columnar structure bodies arranged to match with the plurality of wells,
> a liquid droplet of the cell suspension or the cell adhesive is formed on the holding surface of each of the columnar structure bodies, and
> each of the liquid droplets is contacted with the bottom surface of each of the plurality of wells in a batch treatment.

Supplementary Note 23

The pattern forming method according to any one of Supplementary Notes 16 to 22, comprising:

> forming a liquid droplet of the cell adhesive on the holding surface;
> making the liquid droplet of the cell adhesive contact with the culture surface;
> seeding cells on the culture surface; and
> culturing the cells on the culture surface.

## Claims

1. A pattern forming device for forming a cell pattern or a cell adhesive pattern on a culture surface of a culture base material, the pattern forming device comprising:
   at least one columnar structure body having at least one holding surface for holding a liquid at a tip thereof.

2. The pattern forming device according to claim 1, wherein the holding surface is made of a material having a contact angle of 45° or more, where the contact angle is formed between the holding surface and water.

3. The pattern forming device according to claim 1, wherein the holding surface is made of a resin.

4. The pattern forming device according to claim 1, wherein the holding surface is made of a material that is resistant to autoclaving processes.

5. The pattern forming device according to claim 1, wherein the holding surface is made of a material having weakly hydrophilic or hydrophobicity.

6. The pattern forming device according to claim 1, wherein the columnar structure body has at least one side surface, where an angle formed between the side surface and the holding surface is 5° or more and 175° or less.

7. The pattern forming device according to claim 1, wherein the columnar structure body has at least one side surface, where an angle formed between the side surface and the holding surface is less than 90°.

8. The pattern forming device according to claim 1, further comprising:
   at least one adjustment structure for adjusting a distance between the holding surface and the culture surface.

9. The pattern forming device according to claim 8, wherein the adjustment structure has a through-hole into which the columnar structure body is inserted.

10. The pattern forming device according to claim 1, further comprising:
    at least one position adjustment part for determining a relative position between the culture base material and the columnar structure body in a direction parallel to the culture surface.

11. The pattern forming device according to claim 10, wherein the position adjustment part has a through-hole into which the columnar structure body is inserted.

12. The pattern forming device according to claim 10, wherein a center line of the position adjustment part coincides with a center line of the columnar structure body.

13. The pattern forming device according to claim 1,

    > wherein having a gap of 0.05 mm or more and 5 mm or less with respect to the culture substrate,
    > the gap is connected from the culture surface to the atmosphere.

14. The pattern forming device according to claim 1, wherein the columnar structure body is surrounded by a bank structure.

15. The pattern forming device according to claim 1, further comprising:

    > a substrate; and
    > a plurality of the columnar structure bodies arranged on a surface of the substrate.

16. A pattern forming method of forming the cell pattern or the cell adhesive pattern on the culture surface by using the pattern forming device according to claim 1, the pattern forming method comprising:

> forming a liquid droplet of a cell suspension or a cell adhesive on the holding surface; and making the liquid droplet contact with the culture surface.

17. The pattern forming method according to claim 16, wherein the liquid droplet is formed by dropping the cell suspension or the cell adhesive onto the holding surface in a state where the holding surface faces upward in a vertical direction.

18. The pattern forming method according to claim 17, wherein the liquid droplet is formed so that a gas-liquid-solid interface is formed on an outer edge of the holding surface.

19. The pattern forming method according to claim 17, wherein the liquid droplet is contacted with the culture surface in the state where the holding surface on which the liquid droplet is formed faces upward in the vertical direction.

20. The pattern forming method according to claim 16, wherein a size of the cell pattern or the cell adhesive pattern, which is formed on the culture surface, is controlled by an amount of the liquid droplet.

21. The pattern forming method according to claim 16, wherein a size of the cell pattern or the cell adhesive pattern, which is formed on the culture surface, is controlled by a distance between the holding surface and the culture surface in a case where the liquid droplet is contacted with the culture surface.

22. The pattern forming method according to claim 16,

> wherein the culture surface is a bottom surface of each of a plurality of wells provided in a well plate,
> the pattern forming device has a plurality of the columnar structure bodies arranged to match with the plurality of wells,
> a liquid droplet of the cell suspension or the cell adhesive is formed on the holding surface of each of the columnar structure bodies, and
> each of the liquid droplets is contacted with the bottom surface of each of the plurality of wells in a batch treatment.

23. The pattern forming method according to claim 16, comprising:

> forming a liquid droplet of the cell adhesive on the holding surface;
> making the liquid droplet of the cell adhesive into contact with the culture surface;
seeding cells on the culture surface; and
culturing the cells on the culture surface.

# FIG. 1

# FIG. 2

# FIG. 3

# FIG. 4A

# FIG. 4B

# FIG. 4C

# FIG. 4D

# FIG. 4E

# FIG. 4F

# FIG. 4G

# FIG. 5A

# FIG. 5B

# FIG. 6

## FIG. 7A

## FIG. 7B

# FIG. 8

# FIG. 9

# FIG. 10A

12

17

# FIG. 10B

C

12

17

11

# FIG. 11

# FIG. 12

# FIG. 13

# FIG. 14

# FIG. 15A

# FIG. 15B

## FIG. 16

## FIG. 17

# FIG. 18

# FIG. 19

Europäisches
Patentamt

European
Patent Office

Office européen
des brevets

# EUROPEAN SEARCH REPORT

Application Number

EP 23 20 6144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/298314 A1 (LYONS ALAN M [US] ET AL) 19 October 2017 (2017-10-19)<br>* paragraph [0082] – paragraph [0084] *<br>* paragraphs [0089], [0106], [0117], [0136], [0139], [0143] *<br>* paragraphs [0164], [0185], [0186], [0201] *<br>* paragraph [0213] – paragraph [0215] *<br>* figures 1,2A,2B,4, 33,34,36,44-46,54, 58A,58B * | 1-9, 14-23 | INV.<br>C12M1/32<br>C12M1/12<br>C12M1/30 |
| X | US 2012/187082 A1 (MCGEEHAN JOHN K [US]) 26 July 2012 (2012-07-26)<br>* paragraphs [0004], [0005], [0016], [0017] *<br>* paragraph [0033] – paragraph [0036] *<br>* paragraph [0051] – paragraph [0053] *<br>* claim 17 *<br>* figures 1,6,7 * | 1,8-13, 15 | |
| X,D | JP 2014 168450 A (NAT INST OF ADV IND & TECHNOL) 18 September 2014 (2014-09-18)<br><br>* paragraph [0028] – paragraph [0029] *<br>* paragraph [0032] – paragraph [0037] *<br>* figures 1-9 * | 1,5,8, 10,15, 16,23 | **TECHNICAL FIELDS SEARCHED (IPC)**<br><br>C12M |

The present search report has been drawn up for all claims

| Place of search | Date of completion of the search | Examiner |
|---|---|---|
| Berlin | 19 March 2024 | Cubas Alcaraz, Jose |

EPO FORM 1503 03.82 (P04C01)

## ANNEX TO THE EUROPEAN SEARCH REPORT
## ON EUROPEAN PATENT APPLICATION NO.

EP 23 20 6144

19-03-2024

| Patent document cited in search report | Publication date | Patent family member(s) | Publication date |
|---|---|---|---|
| US 2017298314 A1 | 19-10-2017 | NONE | |
| US 2012187082 A1 | 26-07-2012 | AU 2012207545 B2 | 14-01-2016 |
| | | BR 112013020061 A2 | 25-10-2016 |
| | | CA 2825274 A1 | 26-07-2012 |
| | | CN 103460130 A | 18-12-2013 |
| | | DK 2666056 T3 | 20-01-2020 |
| | | EP 2666056 A1 | 27-11-2013 |
| | | HU E047002 T2 | 28-04-2020 |
| | | IL 227560 A | 31-05-2016 |
| | | JP 5926288 B2 | 25-05-2016 |
| | | JP 2014511108 A | 08-05-2014 |
| | | MX 343449 B | 07-11-2016 |
| | | PL 2666056 T3 | 30-04-2020 |
| | | PT 2666056 T | 17-01-2020 |
| | | US 2012187082 A1 | 26-07-2012 |
| | | WO 2012099787 A1 | 26-07-2012 |
| JP 2014168450 A | 18-09-2014 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014168450 A
- JP 2008079517 A
- JP 2020195385 A